# EUROPEAN PATENT APPLICATION

(11) **EP 4 397 411 A1**
(43) Date of publication of application: **10.07.2024**
(21) Application number: 23155932.9
(22) Date of filing: 10.02.2023
(51) Int. Cl.: B01L 3/00, B01L 7/00, C12Q 1/68, C12N 15/10

(54) **DISPOSABLE PORTABLE LAMP DETECTION MAGNETIC BOX AND ITS CONSTANT TEMPERATURE HEATING DEVICE**

(30) Priority: 06.01.2023 US 202318093901
(71) Applicant: Anbio Biotechnology Limited, Road Town VG1110 Tortola (VG); Wang, Jincheng, Xiamen City Fujian (CN)
(72) Inventor: WANG, Daming, Xiamen City, Fujian Province (CN); WANG, Jincheng, Xiamen City, Fujian Province (CN)
(74) Representative: Sach, Greg Robert

(57) **Abstract**

The present application provides a disposable portable LAMP detection magnetic box, comprising a box body and a detection mechanism, which comprises a first pipeline opened in the box body downward from the top end of the box body, a second pipeline connected to the first pipeline, and a nucleic acid extraction component arranged in the second pipeline to extract the nucleic acid of the nasopharyngeal swab, a third pipeline communicated with the second pipeline and has a reaction solution therein, and an isolation component for isolating the nucleic acid and the reaction solution, wherein the third pipeline is a transparent pipeline, and its peripheral side walls are exposed outside the box body. The present application also provides a constant temperature heating device for a disposable portable LAMP detection magnetic box, comprising a housing, a docking slot opened downward along the top end of the housing for inserting the magnetic box, and a heating component arranged in the housing for heating the magnetic box. The present application has the advantages of light and simple overall structure, easy to carry, test results can be read directly, does not require multiple pipetting steps, and saves time and material costs.

## Description

### TECHNICAL FIELD

The present application relates to the technical field of LAMP detection, in particular to a disposable portable LAMP detection magnetic box and a constant temperature heating device thereof.

### BACKGROUND

Loop-Mediated Isothermal DNA Amplification technology, also known as LAMP technology, has been widely known in the existing technology and is used to develop diagnostic tests for detecting parasites in biological samples.

Since the time and cost of implementing PCR (Polymerase Chain Reaction) technology still prevent conventional laboratories from using PCR technology on a large scale, LAMP technology is increasingly used instead of PCR technology.

LAMP detection is performed under isothermal conditions, which can be maintained in different instruments, such as thermal cyclers and water baths, or the LAMP detection setup employed here. The device enables the amplification of DNA/cDNA of a sample to detect pathogens by heating the detection chamber inside the device.

The existing LAMP detection device has the following problems: 1. It is not convenient to carry, and there is a phenomenon of repeated use, which affects the detection result; 2. Multiple pipetting steps are required, and the time cost and material cost are large; 3. The test result cannot be read directly out.

In order to overcome the above-mentioned technical problems, the inventor has designed a disposable portable LAMP detection magnetic box and its constant temperature heating device, and this application arises at this point.

### SUMMARY OF THE APPLICATION

In order to solve the above problems, the technical solution of the present application is as follows:
A disposable portable LAMP detection magnetic box, comprising a box body and a detection mechanism arranged in the box body, the detection mechanism comprises a first pipeline opened in the box body downward from the top end of the box body along the length direction of the box body and used for putting a nasopharyngeal swab of a patient, a second pipeline connected to an end of the first pipeline away from the top end of the box body, and a nucleic acid extraction component arranged in the second pipeline to extract the nucleic acid of the nasopharyngeal swab of the patient, a third pipeline communicated with the other end of the second pipeline and has a reaction solution therein, and an isolation component arranged between the second pipeline and the third pipeline and used for isolating the nucleic acid and the reaction solution, wherein the third pipeline is fixed to the box body along the width direction of the box body, and the third pipeline is a transparent pipeline, and its peripheral side walls are exposed outside the box body.

It is further configured that: the third pipeline is thermoplastically molded with an acrylic plate.

It is further configured that: the nucleic acid extraction assembly comprises lysate, and the lyse is placed in the second pipeline, the nasopharyngeal swab enters the second pipeline from the first pipeline and is soaked in the lysate.

It is further configured that: the nucleic acid extraction component further comprises several magnetic beads evenly distributed in the lysate.

It is further configured that: the isolation component comprises a control element for controlling whether the second pipeline and the third pipeline are communicated, and a blocking member that prevents the control element from making the second pipeline communicate with the third pipeline, wherein the control member comprises a blocking plate covering the cross-section of the junction of the first pipeline and the third pipeline, and a strip frame for fixing the blocking plate, and a push button fixed on the outer wall of the strip frame and penetrating the outer wall of the box body, the blocking plate is fixed on the inner side wall of the strip frame.

It is further configured that: the blocking member comprises two fixed rods fixed in the box body and parallel to each other, a limiting groove formed between the two fixed rods arranged in parallel, and a blocking rod fixed in the limiting groove, both of the two fixing rods have a slope and a height difference to form an upper locking groove, an upper fixing block and a lower fixing block are respectively fixed at both ends of the blocking rod, the upper fixing block is fixed in the upper locking groove, the lower fixing block is fixed on the outer wall of the strip frame, and is arranged opposite to the pushing button.

It is further configured that: one side of the box body positioned at the third pipeline is provided with a fixing groove, and a magnetic bar is fixed in the fixing groove.

A constant temperature heating device for a disposable portable LAMP detection magnetic box, comprises a housing, a docking slot opened downwards along the top end of the housing and used for inserting the magnetic box, and a heating component arranged in the housing and used for heating the magnetic box.

It is further configured that: the heating component comprises a heater fixed in the housing and close to both sides of the box body, a temperature sensor used for sensing the temperature at the side wall of the box body and transmitting signals, and a temperature display lamp arranged on the outer wall of the housing and used to receive the temperature signal from the temperature sensor, wherein when the temperature received by the temperature display lamp reaches the set temperature, the temperature display lamp lights up in green; when the temperature received by the temperature display lamp does not reach the set temperature, the temperature display lamp lights up in red.

The beneficial effects of the present application are as follows:
1. By setting the box body and the detection mechanism, the detection mechanism comprises the first pipeline, the second pipeline, the third pipeline, the nucleic acid extraction component, and the isolation component. During detection, the patient's nasopharyngeal swab is put into the second pipeline from the first pipeline, and soaked with the nucleic acid extraction component. The nucleic acid contained in the nasopharyngeal swab is incorporated into the nucleic acid extraction component, and the isolation component is driven to release the isolation between the second pipeline and the third pipeline. The nucleic acid enters the reaction solution in the third pipeline for reaction. The third pipeline is a transparent pipeline, and the color displayed in the reaction solution can be seen, and the detection result can be obtained directly according to the color in the reaction solution. Therefore, there is no need for multiple pipetting steps, saving time and material costs, and the overall structure is light and simple, easy to carry.
2. By adding magnetic beads into the lysate and fixing the magnetic bar in the fixing groove on one side of the third pipeline, the patient's nasopharyngeal swab enters the lysate and reacts with the magnetic beads and the lysate. After standing still for about 10 seconds, the isolation component is driven to release the isolation between the second pipeline and the third pipeline. The mixed liquid after the nasopharyngeal swab reacts with the magnetic beads and the lysate enters the third pipeline to react with the reaction liquid (LAMP liquid). The magnetic rod attracts the magnetic beads, which has a certain stirring effect, speeds up the reaction speed, and makes the reaction more complete.
3. By setting a constant temperature heating device for a disposable portable LAMP detection magnetic box, the box body of the magnetic box is heated at a constant temperature. After ensuring that the nasopharyngeal swab reacts with the magnetic beads and the lysate, when the mixed solution enters the third pipeline and reacts with the reaction solution (LAMP solution), the activity of each substance accelerates the reaction speed and makes the reaction more complete.

### BRIEF DESCRIPTION OF THE DRAWINGS

The drawings described here are used to provide a further understanding of the present application and constitute a part of the application. The schematic embodiments and descriptions of the application are used to explain the application and do not constitute an improper limitation to the application, wherein
Fig. 1 is a schematic diagram of the overall structure of embodiment 1 according to the present application;
Fig. 2 is a diagram of the internal structure of the housing in embodiment 1 according to the present application;
Fig. 3 is a connection relationship diagram between the first pipeline, the second pipeline and the third pipeline in Embodiment 1 according to the present application;
Fig. 4 is a schematic diagram of the overall structure when the magnetic box is inserted into the heating device in embodiment 2 according to the present application;
Fig. 5 is a diagram of the overall structure of the heating device in embodiment 2 according to the present application;
Fig. 6 is a diagram of the internal structure of the housing in in embodiment 2 according to the present application

### Reference signs:

1. Box body; 2. Detection mechanism; 21. First pipeline; 22. Second pipeline; 23. Nucleic acid extraction component; 231. lysate; 232. Magnetic beads; 24. Third pipeline; 25. Isolation component; 251. Control element; 2511. Blocking plate; 2512. Strip frame; 2513. Push button; 252. Blocking member; 2521. Fixed rod; 2522, Limiting groove; 2523. Blocking rod; 2524. Upper locking groove; 2525. Upper fixing block; 2526. Lower fixing block; 3. Fixing groove; 4. Magnetic bar; 5. Housing; 6. Docking slot; 7. Heating component; 71. Heater; 72. Temperature sensor; 73. Temperature display lamp.

### DETAILED DESCRIPTION

In order to make the technical problems, technical solutions and beneficial effects to be solved in the present application clearer and clearer, the present application will be further described in detail below in conjunction with the accompanying drawings and embodiments. It should be understood that the specific embodiments described here are only used to explain the present application, not to limit the present application.

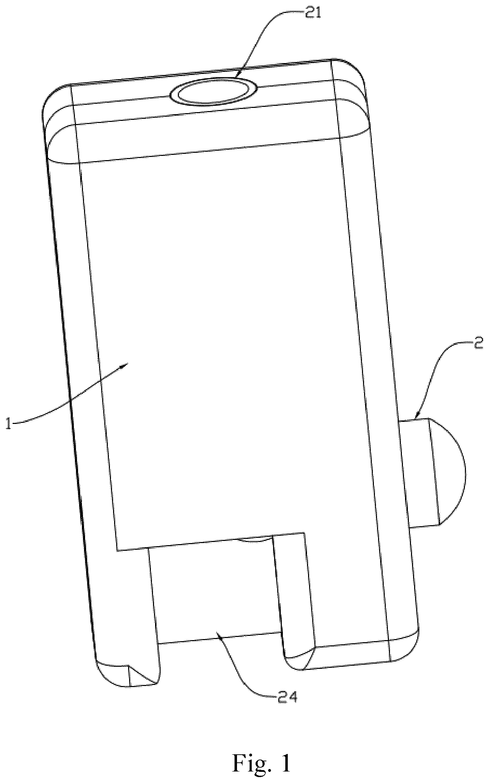

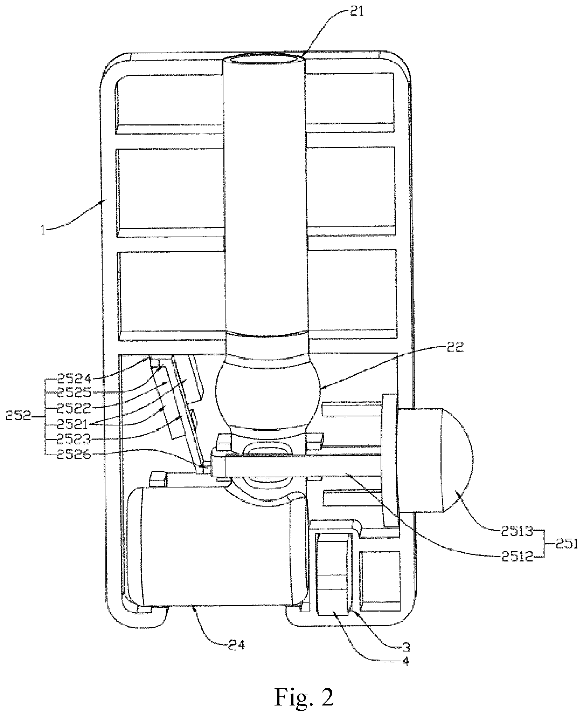

Embodiment 1 is as follows:
Referring to Figures 1 to 3, A disposable portable LAMP detection magnetic box, comprising a box body 1 and a detection mechanism 2 arranged in the box body 1, the detection mechanism 2 comprises a first pipeline 21 opened in the box body 1 downward from the top end of the box body 1 along the length direction of the box body 1 and used for putting a nasopharyngeal swab of a patient, a second pipeline 22 connected to an end of the first pipeline 21 away from the top end of the box body 1, and a nucleic acid extraction component 23 arranged in the second pipeline 22 to extract the nucleic acid of the nasopharyngeal swab of the patient, a third pipeline 24 communicated with the other end of the second pipeline 22 and has a reaction solution therein, and an isolation component 25 arranged between the second pipeline 22 and the third pipeline 24 and used for isolating the nucleic acid and the reaction solution, wherein the third pipeline 24 is fixed to the box body 1 along the width direction of the box body 1, and the third pipeline 24 is a transparent pipeline, and its peripheral side walls are exposed outside the box body 1.

During detection, the patient's nasopharyngeal swab is put into the second pipeline 22 from the first pipeline 21, and soaked with the nucleic acid extraction component 23. The nucleic acid contained in the nasopharyngeal swab is incorporated into the nucleic acid extraction component 23, and the isolation component 25 is driven to release the isolation between the second pipeline 22 and the third pipeline 24. The nucleic acid enters the reaction solution in the third pipeline 24 for reaction. The third pipeline 24 is a transparent pipeline, and the color displayed in the reaction solution can be seen, and the detection result can be obtained directly according to the color in the reaction solution. Therefore, there is no need for multiple pipetting steps, saving time and material costs, and the overall structure is light and simple, easy to carry.

The nucleic acid extraction component 23 (not shown in the figure) comprises lysate 231 (not shown in the figure), and a number of magnetic beads 232 (not shown in the figure) evenly distributed in the lysate 231, and the lysate 231 is placed in the second pipeline 22, the nasopharyngeal swab enters the second pipeline 22 from the first pipeline 21 and is soaked in the lysate 231. One side of the box body 1 positioned at the third pipeline 24 is provided with a fixing groove 3, and a magnetic bar 4 is fixed in the fixing groove 3.

The patient's nasopharyngeal swab enters the lysate 231 and reacts with the magnetic beads 232 and the lysate 231. After standing still for about 10 seconds, the isolation component 25 is driven to release the isolation between the second pipeline 22 and the third pipeline 24. The mixed liquid after the nasopharyngeal swab reacts with the magnetic beads 232 and the lysate 231 enters the third pipeline 24 to react with the reaction liquid (LAMP liquid). The magnetic rod 4 attracts the magnetic beads, which has a certain stirring effect, speeds up the reaction speed, and makes the reaction more complete.

The isolation component 25 comprises a control element 251 for controlling whether the second pipeline 22 and the third pipeline 24 are communicated, and a blocking member 252 that prevents the control element 251 from making the second pipeline 22 communicate with the third pipeline 24, wherein the control member 251 comprises a blocking plate 2511 covering the cross-section of the junction of the first pipeline 21 and the third pipeline 24, and a strip frame 2512 for fixing the blocking plate 2511, and a push button 2513 fixed on the outer wall of the strip frame 2512 and penetrating the outer wall of the box body 1, the blocking plate 2511 is fixed on the inner side wall of the strip frame 2512.

The blocking member 252 comprises two fixed rods 2521 fixed in the box body 1 and parallel to each other, a limiting groove 2522 formed between the two fixed rods 2521 arranged in parallel, and a blocking rod 2523 fixed in the limiting groove 2522, both of the two fixing rods 2521 have a slope and a height difference to form an upper locking groove (2524), an upper fixing block 2525 and a lower fixing block 2526 are respectively fixed at both ends of the blocking rod 2523, the upper fixing block 2525 is fixed in the upper locking groove 2524, the lower fixing block 2526 is fixed on the outer wall of the strip frame 2512, and is arranged opposite to the pushing button 2513.

The patient's nasopharyngeal swab enters the lysate 231 and reacts with the magnetic beads 232 and the lysate 231. After standing still for about 10 seconds, press the push button 2513 to drive the strip frame 2512 to move away from the push button 2513. The strip frame 2512 forms a force on the blocking rod 2523 in a direction away from the push button 2513 through the lower fixing block 2526. The upper fixing block 2525 is fixed in the locking groove to fix the blocking rod 2523 so that the blocking rod 2523 is broken. Along with the strip frame 2512, the blocking plate 2511 removes the coverage of the cross-section of the junction of the first pipeline 21 and the third pipeline 24, so that the mixed solution after the nasopharyngeal swab reacts with the magnetic beads 232 and the lysate 231 enters the third pipeline 24, the breakage of the blocking rod 2523 is an irreversible breakage, thereby ensuring the one-time use of the magnetic box and the accuracy of the detection result.

### The working process of Embodiment 1:

During detection, the patient's nasopharyngeal swab is put into the second pipeline 22 from the first pipeline 21, and soaked with the nucleic acid extraction component 23. The nucleic acid contained in the nasopharyngeal swab is incorporated into the lysate 231, and reacts with the magnetic beads 232 and the lysate 231. After standing still for about 10 seconds, the isolation component 25 is driven to release the isolation between the second pipeline 22 and the third pipeline 24. Nucleic acid enters the reaction solution (liquid containing LAMP) in the third pipeline 24 to react. The third pipeline 24 is a transparent pipeline, and the color displayed in the reaction solution (liquid containing LAMP) can be seen, and the detection result can be obtained directly according to the color in the reaction solution (liquid containing LAMP). Therefore, there is no need for multiple pipetting steps, saving time and material costs, and the overall structure is light and simple, easy to carry.

### Embodiment 2

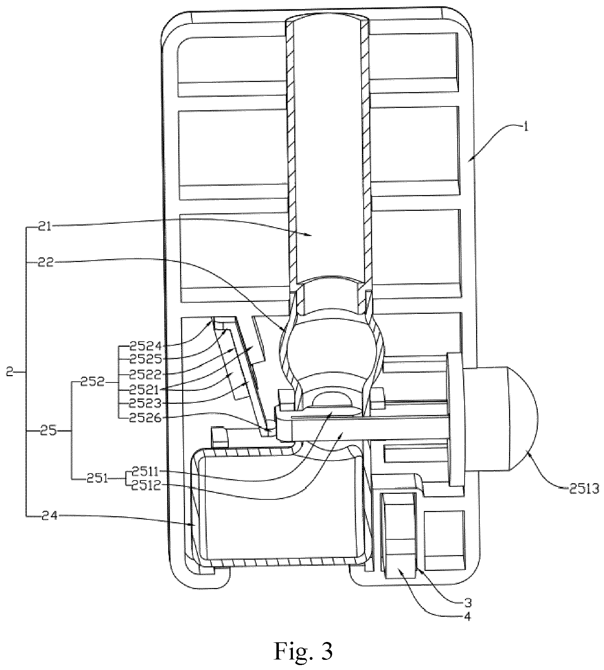

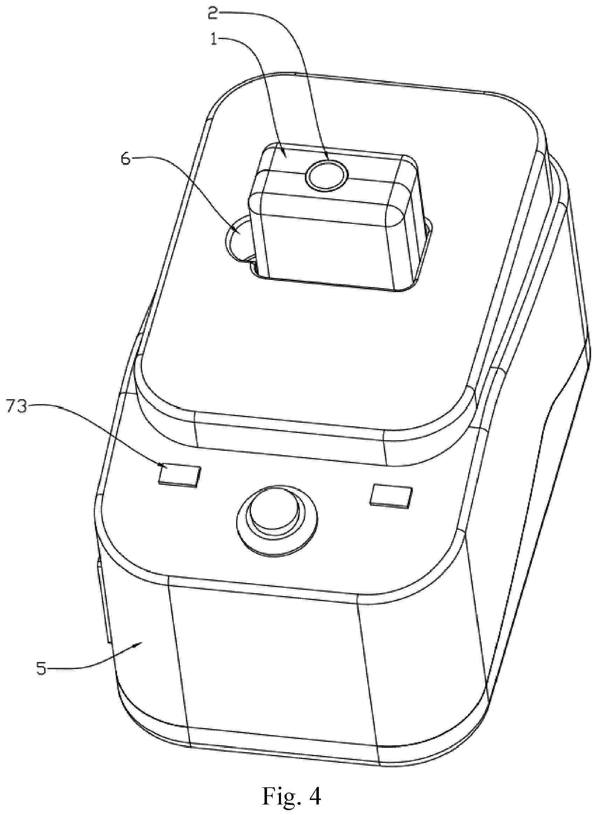

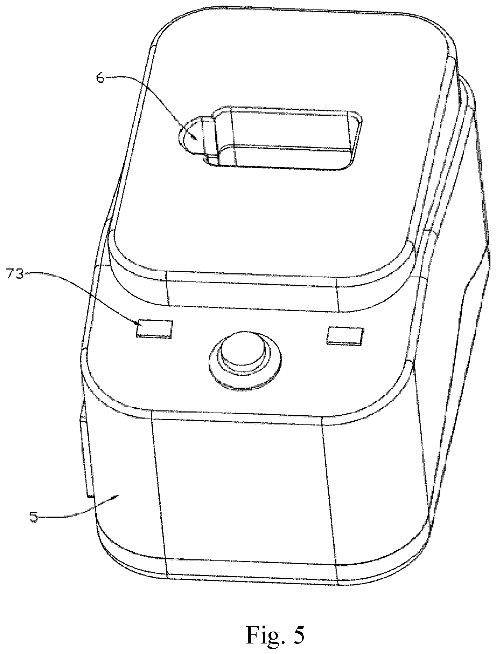

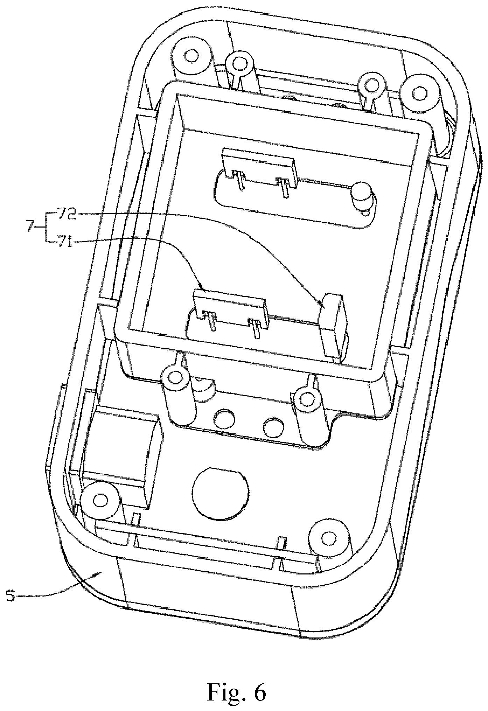

Referring to Fig. 3 to Fig. 6, a constant temperature heating device for a disposable portable LAMP detection magnetic box, comprises a housing 5, a docking slot 6 opened downwards along the top end of the housing 5 and used for inserting the magnetic box, and a heating component 7 arranged in the housing 5 and used for heating the magnetic box.

The heating component 7 comprises a heater 71 fixed in the housing 5 and close to both sides of the box body 1, a temperature sensor 72 used for sensing the temperature at the side wall of the box body 1 and transmitting signals, and a temperature display lamp 73 arranged on the outer wall of the housing 5 and used to receive the temperature signal from the temperature sensor 72. The constant heating temperature of heater 71 is 65 °C, and the display temperature that temperature display lamp 73 sets is 65 °C, wherein when the temperature received by the temperature display lamp 73 reaches the set temperature, the temperature display lamp 73 lights up in green; when the temperature received by the temperature display lamp 73 does not reach the set temperature, the temperature display lamp 73 lights up in red.

### The working principle of Embodiment 2:

The box body of the magnetic box 1 is heated at a constant temperature. After ensuring that the nasopharyngeal swab reacts with the magnetic beads 232 and the lysate 231, when the mixed solution enters the third pipeline 24 and reacts with the reaction solution (LAMP solution), the activity of each substance accelerates the reaction speed and makes the reaction more complete.

The present application has been described as an example in conjunction with the accompanying drawings. Obviously, the specific implementation of the present application is not limited by the above methods. As long as various insubstantial improvements are made using the method concept and technical solution of the present application, or the concept and technical solution of the present application are directly applied to other occasions without improvement, they are all within the protection scope of the application.

## Claims

1. A disposable portable LAMP detection magnetic box, comprising a box body (1) and a detection mechanism (2) arranged in the box body (1), the detection mechanism (2) comprises a first pipeline (21) opened in the box body (1) downward from the top end of the box body (1) along the length direction of the box body (1) and used for putting a nasopharyngeal swab of a patient, a second pipeline (22) connected to an end of the first pipeline (21) away from the top end of the box body (1), and a nucleic acid extraction component (23) arranged in the second pipeline (22) to extract the nucleic acid of the nasopharyngeal swab of the patient, a third pipeline (24) communicated with the other end of the second pipeline (22) and has a reaction solution therein, and an isolation component (25) arranged between the second pipeline (22) and the third pipeline (24) and used for isolating the nucleic acid and the reaction solution, wherein the third pipeline (24) is fixed to the box body (1) along the width direction of the box body (1), and the third pipeline (24) is a transparent pipeline, and its peripheral side walls are exposed outside the box body (1).

2. The disposable portable LAMP detection magnetic box according to claim 1, wherein the third pipeline (24) is thermoplastically molded with an acrylic plate.

3. The disposable portable LAMP detection magnetic box according to claim 1, wherein the nucleic acid extraction assembly (23) comprises lysate (231), and the lyse (231) is placed in the second pipeline (22), the nasopharyngeal swab enters the second pipeline (22) from the first pipeline (21) and is soaked in the lysate (231).

4. The disposable portable LAMP detection magnetic box according to claim 3, wherein the nucleic acid extraction component (23) further comprises several magnetic beads (232) evenly distributed in the lysate (231).

5. The disposable portable LAMP detection magnetic box according to claim 1, wherein the isolation component (25) comprises a control element (251) for controlling whether the second pipeline (22) and the third pipeline (24) are communicated, and a blocking member (252) that prevents the control element (251) from making the second pipeline (22) communicate with the third pipeline (24), wherein the control member (251) comprises a blocking plate (2511) covering the cross-section of the junction of the first pipeline (21) and the third pipeline (24), and a strip frame (2512) for fixing the blocking plate (2511), and a push button (2513) fixed on the outer wall of the strip frame (2512) and penetrating the outer wall of the box body (1), the blocking plate (2511) is fixed on the inner side wall of the strip frame (2512).

6. The disposable portable LAMP detection magnetic box according to claim 5, wherein the blocking member (252) comprises two fixed rods (2521) fixed in the box body (1) and parallel to each other, a limiting groove (2522) formed between the two fixed rods (2521) arranged in parallel, and a blocking rod (2523) fixed in the limiting groove (2522), both of the two fixing rods (2521) have a slope and a height difference to form an upper locking groove (2524), an upper fixing block (2525) and a lower fixing block (2526) are respectively fixed at both ends of the blocking rod (2523), the upper fixing block (2525) is fixed in the upper locking groove (2524), the lower fixing block (2526) is fixed on the outer wall of the strip frame (2512), and is arranged opposite to the pushing button (2513).

7. The disposable portable LAMP detection magnetic box according to claim 4, wherein one side of the box body (1) positioned at the third pipeline (24) is provided with a fixing groove (3), and a magnetic bar (4) is fixed in the fixing groove (3).

8. A constant temperature heating device for a disposable portable LAMP detection magnetic box, comprising a housing (5), a docking slot (6) opened downwards along the top end of the housing (5) and used for inserting the magnetic box, and a heating component (7) arranged in the housing (5) and used for heating the magnetic box.

9. The constant temperature heating device for a disposable portable LAMP detection magnetic box according to claim 8, wherein the heating component (7) comprises a heater (71) fixed in the housing (5) and close to both sides of the box body (1), a temperature sensor (72) used for sensing the temperature at the side wall of the box body (1) and transmitting signals, and a temperature display lamp (73) arranged on the outer wall of the housing (5) and used to receive the temperature signal from the temperature sensor (72), wherein when the temperature received by the temperature display lamp (73) reaches the set temperature, the temperature display lamp (73) lights up in green; when the temperature received by the temperature display lamp (73) does not reach the set temperature, the temperature display lamp (73) lights up in red.
